# FASCICULE DE BREVET EUROPEEN

(11) **EP 1 608 351 B1**
(45) Date de publication et mention de la délivrance du brevet: **06.09.2006**
(21) Numéro de dépôt: 04742296.9
(22) Date de dépôt: 19.03.2004
(51) Int. Cl.: A61K 31/05, A61K 31/20, A61K 31/19, A61P 29/00, A61K 38/44

(54) **COMPOSITIONS RENFERMANT UNE SUPEROXYDE DISMUTASE ET UN INHIBITEUR DE LA 5-LIPOXYGENASE SELECTIONNE ET APPLICATIONS**
ZUSAMMENSETZUNGEN ENTHALTEND EINE SUPEROXIDDISMUTASE, EINEN 5-LIPOXYGENASE-INHIBITOR SOWIE DEREN VERWENDUNG
COMPOSITIONS CONTAINING SUPEROXIDE DISMUTASE AND A SELECTED ET 5-LIPOXYGENASE INHIBITOR AND USES THEREOF

(30) Priorité: 28.03.2003 FR 0303865
(43) Date de publication de la demande: 28.12.2005
(73) Titulaire: Isocell S.A., 75015 Paris (FR)
(72) Inventeur: DUGAS, Bernard, décédé (FR)
(74) Mandataire: Goulard, Sophie
(86) Numéro de dépôt international: PCT/FR2004/000682
(87) Numéro de publication internationale: WO 2004/087119

(56) Documents cités:
- EP-A- 0 366 869
- WO-A-86/02556
- WO-A-92/01437
- US-A- 5 561 052

## Description

La présente Invention est relative à des compositions renfermant au moins une superoxyde dismutase (SOD) et au moins un inhibiteur de la 5-lipoxygénase (5-LO) convenablement sélectionné, ainsi que leurs utilisations, à titre de médicament ou de produit diététique, pour la prévention et/ou le traitement de maladies inflammatoires chroniques d'origine allergique ou non.

Les états inflammatoires se caractérisent, de façon symptomatique, par des douleurs, des rougeurs et des gonflements de l'organe enflammé. Cette pathologie clinique résulte de la libération de médiateurs de l'inflammation, principalement à partir des leucocytes activés qui migrent vers la zone cible. Parmi les médiateurs clé de l'inflammation, on peut en particulier citer les eicosanoïdes polyinsaturés (n-6), la prostaglandine E₂ (PGE₂), les leucotriènes et en particulier le leucotriène B₄ (LTB₄), qui sont des dérivés polyinsaturés (n-6) d'un acide gras particulier qui est l'acide arachidonique (ou éicosanique). D'autres médiateurs importants de l'inflammation sont les cytokines, l'interleukine 1β (IL-1β) et le *Tumor Necrosis Factor* α (TNF-α).

Afin de lutter contre le processus inflammatoire, plusieurs voies d'action peuvent être envisagées ; ces différentes voies pouvant de plus être imbriquées entre elles.

Une des voies permettant de lutter contre le processus inflammatoire est par exemple de diminuer ou d'empêcher la production des leucotriènes et en particulier du LTB₄. La 5-lypoxygénase est une enzyme qui catalyse les deux premières étapes de la synthèse des leucotriènes à partir de l'acide arachidonique (Samuelsson B. *et al*., Science, 1983, **220**, 568-575). L'activité de cette enzyme peut varier en fonction de différents facteurs comme par exemple les quantités d'ions calcium et d'adénosine triphosphate (ATP) dans les cellules (Jackschik BA *et al*., Biochem. Biophys. Res. Commun., 1980, **95,** 103-110 ; Rouzer CA et Samuelsson B, Proc. Natl. Acad. Sci. USA, 1987, **84,** 7393-7397). *In vitro*, la 5-LO est activée dans des conditions qui induisent la peroxydation lipidique (Riendeau *et al*., Biochem. J., 1989, **263**, 565-572). *In vivo,* on observe une augmentation de la formation des espèces activées de l'oxygène au niveau des sites inflammatoires, qui parmi d'autres signaux, sont impliquées dans l'activation de la 5-LO et par la même dans la synthèse subséquente des leucotriènes par les leucocytes activés. Le profil biologique des leucotriènes suggère donc que des molécules aptes à inhiber la synthèse de la 5-LO (inhibiteurs de la 5-LO) peuvent avoir un intérêt thérapeutique pour la prévention et/ou le traitement des phénomènes inflammatoires et allergiques.

Il existe au moins deux grandes classes d'inhibiteurs de la 5-LO, à savoir les inhibiteurs d'origine synthétique (ou chimique) et les inhibiteurs d'origine naturelle.

Parmi les inhibiteurs d'origine synthétique, on peut en particulier citer les dérivés de l'acide hydroxamique ou de la N-hydroxyurée, certains dérivés aromatiques hydroxylés comme l'acide caféique (ou acide 3-(-,4-dihydroxyphényl)-2-propénoïque), l'acide nordihydroguairétique (NDGA), la 5,6,7-trihydroxy-2-phényl-4H-1-benzopyran-4-one (ou Baicalein), la 6,7-dihydroxy-2H-1-benzopyran-2-one (ou Esculetin), la 1,1',6,6',7,7'-hexahydroxy-3,3'-diméthyl-5,5'bis(1-méthyléthyl) [2,2'binaphtalène]-8,8'dicarboxaldéhyde (ou Gossypol), le racémate de l'acide α-méthyl 2-fluoro [1,1'-biphényl]-4-acétique (ou Flurbiprofen), l'α-phényl-3-(2-quinolylméthoxy) benzèneméthanol, etc...

Parmi les inhibiteurs d'origine naturelle, on peut en particulier les acides gras polyinsaturés, notamment les acides gras oméga-3 contenus dans certaines espèces végétales ainsi que dans tous les poissons et crustacés tels que l'acide (3n-3) linolénique, l'acide 5,8,11,14-éicosatetraynoïque, l'acide 5,8,11-éicosatriynoïque, l'acide éicosapentaénoique (4n-3), l'acide docosapentaénoique, l'acide docosahexanéoique et les acides gras oméga-6 contenus dans toutes les huiles végétales et tels que l'acide linoléique (18:2) et ses dérivés. Il existe à cet égard sur le marché un produit anti-inflammatoire issu de la moule verte de Nouvelle-Zélande (*Perna Canaliculus*) qui est un mélange complexe d'acides gras polyinsaturés de type oméga-3 et oméga-6, commercialisé sous la dénomination commerciale LYPRINOL®.

Une autre voie pour lutter contre le processus inflammatoire peut par exemple consister à favoriser la production endogène de molécules anti-inflammatoires. En effet, au cours du processus inflammatoire, les cellules responsables de la libération des médiateurs de l'inflammation (monocytes, macrophages et lymphocytes T), produisent également des médiateurs anti-inflammatoires tels que les cytokines anti-inflammatoires comme par exemple l'interleukine-10 (IL-10) qui est capable d'inhiber la production des cytokines inflammatoires comme l'IL-1β ou l'interleukine-6 (IL-6), ainsi que la production du TNF-α par les macrophages.

Par ailleurs, les superoxyde dismutases (SODs) forment une classe particulière de métalloprotéines contenant du fer, du cuivre, du zinc ou du manganèse, et sont des enzymes capables d'induire la dismutation des anions superoxydes qui sont des radicaux libres également impliqués dans le processus inflammatoire. Du fait de cette propriété, les SODs sont largement utilisées en thérapeutique pour le traitement et/ou la prévention des pathologies induites par les radicaux libres telles que les états inflammatoires.

De nombreux auteurs ont démontré que l'utilisation de ces produits permet de contrôler les différents processus inflammatoires en agissant notamment au niveau des différentes voies métaboliques du processus inflammatoire.

Il est donc important de pouvoir disposer d'un produit anti-inflammatoire apte à prévenir et/ou à traiter les maladies inflammatoires d'origine allergique ou non. Un tel produit doit de façon idéale être capable non seulement de diminuer la production des médiateurs de l'inflammation (LTB₄, TNF-α) et les espèces activées de l'oxygène (EAO), mais également d'induire la production de cytokines anti-inflammatoires telles que l'IL-10.

Or, dans le cadre de leur travaux, les Inventeurs ont démontré que l'association d'au moins une SOD et d'au moins un inhibiteur de 5-LO convenablement sélectionné permet non seulement d'obtenir un effet anti-inflammatoire important mais que cet effet est supérieur à la somme des effets induits individuellement par les SODs et les inhibiteurs de 5-LO ; on observe donc un effet de synergie qui n'est par ailleurs pas obtenu avec tous les inhibiteurs de la 5-LO. C'est cette découverte qui est à la base de la présente Invention.

La présente Invention a donc pour objet une composition pharmaceutique ou diététique, caractérisée par le fait qu'elle renferme, dans un support pharmaceutiquement ou diététiquement acceptable :
- au moins un inhibiteur de la 5-lipoxygénase sélectionné dans le groupe constitué par l'acide nordihydroguairétique (NDGA), le racémate de l'acide α-méthyl 2-fluoro [1,1'-biphényl]-4-acétique (Flurbiprofen) et les acides gras polyinsaturés de la série oméga-3 comportant 18 ou 20 atomes de carbone et au moins 3 liaisons insaturées, et
- au moins une superoxyde dismutase ;
ladite composition étant exempte de dérivés de la pyrimidine 3-oxyde.

La demande internationale WO 92/01437 décrit des compositions destinées à freiner la chute de cheveux à base de dérivés de pyrimidine 3-oxyde à titre de principe actif et pouvant en outre renfermer de la superoxyde dismutase et des acide gras tels que l'acide linoléique et l'acide linolénique. De telles compositions ne font pas partie de l'Invention.

Les Inventeurs ont démontré que, de manière surprenante, lorsqu'on utilise une composition conforme à la présente Invention et telle que définie précédemment, l'effet anti-inflammatoire qui est observé est supérieur à la somme des effets anti-inflammatoires observé avec chacun des composés testés individuellement dans les mêmes conditions.

Selon l'Invention, les acides gras polyinsaturés de la série oméga-3 sont de préférence choisis parmi l'acide linolénique (C18 ; 3n-3), l'acide 5,8,11,14-éicosatetraynoïque, l'acide éicosapentaénoïque (C20, 4n-3), l'acide éicosatriynoïque et leurs mélanges.

Selon une forme de réalisation avantageuse, la composition conforme à l'Invention renferme un mélange d'acide linolénique, d'acide 5,8,11,14-éicosatetraynoïque et d'acide éicosapentaénoique. Un tel mélange est en particulier contenu dans le produit commercial vendu sous la dénomination LYPRINOL®.

Parmi les SODs pouvant être présentes au sein de la composition conforme à l'Invention, on peut en particulier citer les SODs d'origine animale, végétale ou bactérienne telles que par exemple la SOD cuivre/zinc (SOD Cu/Zn) d'origine bovine, la SOD manganèse (SOD-Mn) extraite des mitochondries, la SOD fer (SOD-Fe), les SOD extraites des plantes comme par exemple du melon ou des brocolis, ainsi que les SODs recombinantes d'origine humaine, animale ou végétale.

Selon une forme de réalisation préférée de l'Invention, la ou les SODs utilisées sont combinées à au moins un vecteur apte à faciliter le passage transmembranaire des SODs. Un tel vecteur peut en particulier être choisi parmi les prolamines d'origine végétale telles que par exemple les prolamines de blé (gliadine), de mais (zéine) ou de riz, les céramides et les polypeptides riches en arginine et/ou en lysine.

Selon l'Invention, on entend par polypeptides riches en arginine ou en lysine, des polypeptides dans lesquels l'arginine et/ou la lysine représente au moins 60 à 70 % environ de la teneur total en acides aminés. Parmi ces polypeptides, on peut en particulier citer la polylysine, ainsi que les polypeptides issus par exemple de la protéine Tat du VIN-1.

Des SODs combinées à des prolamines ou à des céramides sont plus particulièrement décrites dans le brevet EP 804 225 au nom de la Demanderesse ; cette combinaison permettant notamment d'augmenter la biodisponibilité de la SOD lorsqu'elle administrée par voie orale.

L'utilisation de polypeptides riches en arginine ou en lysine pour faciliter l'internalisation des protéines dans les cellules est notamment décrite dans l'article de Fawell S. *et al*., Proc. Natl. Acad. Sci. USA, 1994, 91, 664-668.

Au sein de la composition conforme à l'Invention, le rapport pondéral SOD(s)/inhibiteur(s) de la 5-LO est de préférence compris entre 5/1 et 1/1 lorsque le ou les inhibiteurs de la 5-LO sont choisis parmi le NDGA et le Flurbiprofen et entre 7/1 et 1/1 lorsque le ou les inhibiteurs de la 5-LO sont choisis parmi les acides gras polyinsaturés de la série oméga-3 tels que ceux définis précédemment.

Selon une forme de réalisation particulièrement préférée de l'Invention, le rapport pondéral SOD(s)/inhibiteur(s) de la 5-LO est de 2/1 environ lorsque le ou les inhibiteurs de la 5-LO sont choisis parmi le NDGA et le Flurbiprofen et de 3/1 environ lorsque le ou les inhibiteurs de la 5-LO sont choisis parmi les acides gras polyinsaturés de la série oméga-3 tels que ceux définis précédemment.

La composition conforme à l'Invention peut également renfermer un ou plusieurs principes actifs additionnels tels que ceux utilisés dans les compositions pharmaceutiques ou diététiques destinées à prévenir et/ou à traiter les états inflammatoires, et parmi lesquels on peut notamment citer les autres principes actifs anti-inflammatoires, les oligo-éléments, les vitamines, la D-glucosamine, les méthylsulfones, etc...

Le véhicule pharmaceutiquement ou diététiquement acceptable selon l'Invention est de préférence constitué par un ou plusieurs excipients classiquement utilisés pour la préparation de compositions pharmaceutiques ou diététiques, tels que des anti-agglomérants, des anti-oxydants, des colorants, des agents modifiants du goût, des édulcorants, des agents de lissage, de montage, d'isolation et de manière générale, tout excipient classiquement utilisé dans l'industrie pharmaceutique ou diététique.

Bien entendu, l'homme de l'art veillera à cette occasion à ce que le ou les excipients éventuellement utilisés soient compatibles avec les propriétés intrinsèques attachées à la composition objet de la présente Invention.

Selon une forme de réalisation particulière de l'Invention, la composition pharmaceutique ou diététique peut se présenter sous la forme d'un kit comprenant au moins une composition (A) renfermant au moins un inhibiteur de la 5-LO tel que défini précédemment et au moins une composition (B) renfermant au moins une superoxyde dismutase, lesdites compositions (A) et (B) étant destinées à être mélangées ensemble au moment de l'emploi.

La composition pharmaceutique ou diététique conforme à l'Invention est destinée à être administrée par voie orale, et peut être formulée sous diverses formes, telles que sous forme sèche, pâteuse, semi-pâteuse, huileuse, liquide ou semi-liquide. Elle peut donc se présenter sous la forme de comprimés, de gélules, de solutions ou suspensions buvables, de tablettes, ou sous toute autre forme appropriée au mode d'administration.

Lorsqu'elle est destinée à être utilisée en diététique, la composition conforme à l'Invention peut en outre être incorporée à titre d'ingrédient, dans des produits alimentaires comme par exemple des biscuits, des barres énergétiques, des poudres pour boissons, des boissons, etc... L'Invention a donc également pour objet un produit alimentaire renfermant, à titre d'ingrédient, au moins une composition conforme à l'Invention et telle que définie précédemment.

La composition pharmaceutique ou diététique conforme à l'Invention présente les propriétés de pouvoir être notamment utilisée pour la prévention et/ou le traitement des états inflammatoires chroniques d'origine allergique ou non.

La présente Invention a donc également pour objet l'utilisation d'au moins une composition telle que définie précédemment à titre de médicament, et en particulier à titre de médicament destiné à la prévention et/ou au traitement des états inflammatoires chroniques, d'origine allergique ou non.

Dans ce cas, la composition pharmaceutique conforme à l'Invention est de préférence administrée à des doses permettant de délivrer de 10 à 20 mg/jour/kg de SOD environ et de 30 à 60 mg/jour/kg d'inhibiteur de la 5-LO.

La présente Invention a également pour objet l'utilisation d'au moins une composition telle que définie précédemment à titre de produit diététique, et en particulier à titre de produit diététique (ou neutraceutique) destiné à la prévention des états inflammatoires chroniques, d'origine allergique ou non.

Dans ce cas, la composition conforme à l'Invention est de préférence administrée à des doses permettant de délivrer de 5 à 10 mg/jour/kg de SOD environ et de 15 à 30 mg/jour/kg d'inhibiteur de la 5-LO (supplémentation).

Enfin, l'Invention a pour objet l'utilisation d'au moins une composition telle que définie précédemment pour la préparation d'un médicament destiné à la prévention et/ou traitement des états inflammatoires chroniques d'origine allergique ou non ou pour la préparation d'un produit diététique (ou nutraceutique) destiné à la prévention des états inflammatoires chroniques d'origine allergique ou non.

Outre les dispositions qui précèdent, l'Invention comprend encore d'autres dispositions qui ressortiront de la description qui va suivre, qui se réfère à un exemple décrivant l'étude de l'effet anti-inflammatoire (par la quantification de la production d'IL-10 endogène) d'une association de SOD-Cu/Zn bovine combinée à un polypeptide issu de la protéine Tat du VIH-1 et de NDGA ou de LYPRINOL® comparativement à chacun de ces composés testés individuellement, et à un exemple décrivant l'étude de l'effet anti-inflammatoire (par la quantification de la production d'IL-10 endogène) d'une association de SOD-Cu/Zn combinée à un polypeptide issu de la protéine Tat du VIH-1 et de différents acides gras polyinsaturés ou d'inhibiteurs chimiques de la 5-LO comparativement à chacun de ces composés testés individuellement.

Il doit être bien entendu toutefois que ces exemples sont donnés uniquement à titre d'illustration de l'objet de l'Invention, dont ils ne constituent en aucune manière une limitation.

### EXEMPLE 1 : ETUDE COMPARATIVE DE L'EFFET ANTI-INFLAMMATOIRE (PAR LA PRODUCTION D'IL-10 ENDOGENE) D'UNE ASSOCIATION DE SOD Cu/Zn BOVINE COMBINEE A UN POLYPEPTIDE ET DE NDGA OU DE LYPRINOL®

### 1) Principe de l'étude

Des monocytes humains sont d'abord préactivés par des composés capables d'induire un état inflammatoire :
- soit par l'intermédiaire de l'interféron-γ pour induire une inflammation de type Th 1 (production d'IFN-γ par les lymphocytes T),
- soit par l'intermédiaire de l'interleukine-4 (IL-4) pour induire une inflammation de type Th2 (production d'IL-4 par les lymphocytes T).

Les cellules ainsi préactivées sont ensuite stimulées respectivement par des lipopolysaccharides (LPS) bactériens ou par un anticorps monoclonal anti-CD23 (clone 135 fourni par l'Hôpital Notre Dame, Montréal, Canada ; immunoglobulines G1 (IgG1) de souris) qui reconnaît spécifiquement le récepteur de basse affinité pour les IgE ; afin de déclencher le processus inflammatoire.

Les monocytes sont ensuite mis en contact avec les composés anti-inflammatoires à tester (SODs et inhibiteurs de la 5-LO) de façon à en évaluer les propriétés anti-inflammatoires par le biais de la mesure de la quantité d'anti-inflammatoire produit de façon endogène par les cellules (quantité d'IL-10).

### 2) Matériel et méthode

En pratique, des monocytes humains normaux (10⁶ cellules/ml), obtenus après adhérence des cellules mononucléées circulantes de patients normaux, c'est-à-dire non allergiques et ne présentant aucun signe clinique traduisant un état inflammatoire, sont cultivées pendant 24 heures dans un milieu de culture Dubelco Eagle Modified (DEM) additionné de 1 % en poids de sérum de veau foetal (SVF) sans endotoxine (Gibco, France), de 10 µg/ml d'une solution de streptomycine à 5 %, et de 10 mM de pyruvate de sodium, en présence de 30 ng/ml d'IFN-γ ou d'IL-4.

Après culture, les cellules activées sont ensuite reprises dans du milieu frais à raison de 5.10⁵ cellules par ml.

Les cellules qui ont été activées par l'IFN-γ sont ensuite stimulées par 1 ng/ml de lipopolysaccharide d'*E. Coli* vendu sous la dénomination commerciale LPS par la société SIGMA.

Les cellules qui ont été activées par l'IL-4 sont ensuite stimulées par 10 µg/ml d'anticorps anti-CD23 clone 135.

Les cultures de cellules sont alors mises en contact avec les composés anti-inflammatoires :
- SOD-Cu/Zn d'origine bovine (vendue par la société SIGMA), combinée au peptide issu de la protéine Tat du VIH-1 (vendu sous la dénomination commerciale Chariot® par la société Active Motif, Belgique) (SOD-vectorisée), en solution dans du milieu de culture, à raison de µg/ml. La SOD vectorisée est préparée en faisant incuber 1 µg de SOD-Cu/Zn bovine en suspension dans du milieu de culture avec 30 µg de polypeptide, pendant 30 minutes à température ambiante.
- NDGA, vendu par la société Calbiochem, à raison de 10 µg/ml,
- Lyprinol®, à raison de 10 µg/ml.
- mélange de SOD-vectorisée et de NDGA en proportions 1/3, à raison de 1 µg/ml pour la SOD-vectorisée et de 3 µg/ml pour le NDGA ou
- mélange de SOD-vectorisée et de Lyprinol® en proportions 1/3, à raison de 1 µg/ml pour la SOD-vectorisée et de 3 µg/ml pour le Lyprinol®.

Par ailleurs, une culture de cellules dans laquelle aucun composé anti-inflammatoire n'a été ajouté a également été réalisée afin de servir de contrôle. Ce contrôle permet de connaître le taux de base de la production endogène d'IL-10 après activation et stimulation.

La quantité de cytokine endogène IL-10 produite par les cellules est évaluée par dosage dans les surnageants de culture 24 heures après la stimulation et la mise en contact avec les anti-inflammatoires à tester, à l'aide d'un kit de dosage commercialisé sous la dénomination Quantikine® Human IL-10 EIA Kit par la société R&D system, (GB) et selon la méthode spécifiée par le fabriquant.

### 3) Résultats

Les résultats obtenus sont reportés dans le tableau 1 ci-après :

**TABLEAU I**

| **Produits testés** | **Quantité de IL-10 (en pg/ml)** | |
|---|---|---|
| | **Activation/Stimulation : IFN-γ/LPS** | **Activation/Stimulation : IL-4/anti-CD23** |
| Contrôle | 200 | 180 |
| SOD-vectorisée * | 290 | 250 |
| NDGA * | 210 | 195 |
| Lyprinol® * | 305 | 300 |
| SOD-vectorisée + NDGA | **690** | **600** |
| SOD-vectorisée + Lyprinol® | **910** | **890** |

| | | |
|---|---|---|
| * résultats comparatifs ne faisant pas partie de l'Invention | | |

Ces résultats montrent que de façon surprenante l'association d'une SOD et de NDGA ou de Lyprinol® a un effet synergique très important sur la production de cytokine IL-10 par les cellules. Cette association permet donc d'induire la production de ce promoteur anti-inflammatoire endogène de façon beaucoup plus importante que lorsque la SOD ou le NDGA ou le Lyprinol® sont utilisés individuellement. Ces résultats sont d'autant plus remarquables que les valeurs obtenues pour le NDGA seul montrent que celui-ci n'a pratiquement pas d'effet sur la production endogène d'IL-10.

### EXEMPLE 2 : ETUDE COMPARATIVE DE L'EFFET ANTI-INFLAMMATOIRE (PAR LA PRODUCTION D'IL-10 ENDOGENE) D'UNE ASSOCIATION DE SOD Cu/Zn BOVINE COMBINEE A UN POLYPEPTIDE ET DE DIFFERENTS INHIBITEURS DE LA 5-LO

Cette étude a été réalisée selon le même protocole que celui décrit ci-dessus à l'exemple 1 sauf que l'activation et la stimulation des cellules ont seulement été effectuées par le couple IL-4/anti-CD23.

Dans cet exemple, trois acides gras polyinsaturés de la série oméga-3 conformes à l'Invention (l'acide linolénique, l'acide 5,8,11,14-éicosatetraynoïque, l'acide éicosapentaénoique et l'acide éicosatriynoique) ainsi que le Flurbiprofen ont été testés, en association ou non avec de la SOD-Cu/Zn vectorisée telle que décrite ci-dessus à l'exemple 1.

A titre comparatif, d'autres inhibiteurs de la 5-LO connus pour leurs propriétés anti-inflammatoires ont également été testés, individuellement et en association avec la SOD-vectorisée (Baicalein, acide caféique, l'acide linoléique, l'acide docosapentaénoique et l'acide docosahexanéoique).

La SOD-vectorisée a été utilisée à raison de 4 µg/ml de culture cellulaire, et les inhibiteurs de la 5-LO ont été utilisés à raison de 10 µg/ml de culture cellulaire.

Les résultats obtenus sur la quantité de IL-10 produite par les cellules sont reportés dans le tableau II ci-après :

A titre de référence, lorsque la SOD-vectorisée est utilisée seule, un taux d'IL-10 de 220 pg/ml de surnageant est obtenu, le taux de référence de la quantité d'IL-10 produite par les cellules dans le surnageant (contrôle) étant de 120 pg/ml.

**TABLEAU II**

| **Produits testés** | **Quantité d'IL-10 (en pg/ml)** | |
|---|---|---|
| | **Composé utilisé seul** | **Composé utilisé avec la SOD-vectorisée** |
| Acide linolénique | 180 | **420 **** |
| Acide 5,8,11,14-éicosatetraynoïque | 190 | **450 **** |
| Acide éicosapentaénoique | 200 | **510 **** |
| Acide éicosatriynoique | 190 | **420 **** |
| Flurbiprofen | 80 | **320 **** |
| Baicalein * | 120 | 230 |
| Acide caféique * | 120 | 190 |
| Acide linoléique * | 150 | 300 |
| Acide docosapentaénoique * | 120 | 210 |
| Acide docosahexanéoique * | 130 | 210 |

| | | |
|---|---|---|
| * Inhibiteur de la 5-LO ne faisant pas partie de l'Invention | | |
| ** Résultats significatifs de l'effet de synergie entre la SOD-vectorisée et l'inhibiteur de la 5-LO testé (c'est-à-dire que l'effet obtenu avec l'association est supérieur à la somme des effets obtenus avec chacun des composés testé individuellement). | | |

Ces résultats montrent que seules les associations conformes à la présente Invention, c'est-à-dire renfermant de la SOD et un inhibiteur de la 5-LO convenablement sélectionné, permettent d'obtenir un effet de synergie sur la production endogène d'IL-10.

## Revendications

1. Composition pharmaceutique ou diététique, **caractérisée par le fait qu'**elle renferme, dans un support pharmaceutiquement ou diététiquement acceptable :
- au moins un inhibiteur de la 5-lipoxygénase sélectionné dans le groupe constitué par l'acide nordihydroguairétique (NDGA), le racémate de l'acide α-méthyl 2-fluoro [1,1'-biphényl]-4-acétique (Flurbiprofen) et les acides gras polyinsaturés de la série oméga-3 comportant 18 ou 20 atomes de carbone et au moins 3 liaisons insaturées, et
- au moins une superoxyde dismutase ;
ladite composition étant exempte de dérivés de la pyrimidine 3-oxyde.

2. Composition selon la revendication 1, **caractérisée par le fait que** les acide gras polyinsaturés de la série oméga-3 sont choisis parmi l'acide linolénique (C18 ; n-3), l'acide 5,8,11,14-éicosatetraynoïque, l'acide éicosapentaénoique, l'acide éicosatriynoique et leurs mélanges.

3. Composition selon la revendication 2, **caractérisée par le fait qu'**elle renferme un mélange d'acide linolénique, d'acide 5,8,11,14-éicosatetraynoïque et d'acide éicosapentaénoique.

4. Composition selon l'une quelconque des revendications précédentes, **caractérisée par le fait que** les SODs sont choisies parmi les SODs d'origines animale, végétale, bactérienne, et les SODs recombinantes d'origine humaine, animale ou végétale.

5. Composition selon l'une quelconque des revendications précédentes, **caractérisée par le fait que** la ou les SODs sont combinées à au moins un vecteur apte à faciliter le passage transmembranaire des SODs.

6. Composition selon la revendication 5, **caractérisée par le fait que** ledit vecteur est choisi parmi les prolamines d'origine végétale, les céramides et les polypeptides riches en arginine et/ou en lysine.

7. Composition selon la revendication 6, **caractérisée par le fait que** les prolamines végétales sont choisies parmi les prolamines de blé (gliadine), de mais (zéine) et de riz.

8. Composition selon l'une quelconque des revendications précédentes, **caractérisée par le fait que** le rapport pondéral SOD(s)/inhibiteur(s) de la 5-lipoxygénase est compris entre 5/1 et 1/1 lorsque le ou les inhibiteurs de la 5-lipoxygénase sont choisis parmi le NDGA et le Flurbiprofen et entre 7/1 et 1/1 lorsque le ou les inhibiteurs de la 5-lipoxygénase sont choisis parmi les acides gras polyinsaturés de la série oméga-3 tels que définis à l'une quelconque des revendications 1 à 3.

9. Composition selon la revendication 8, **caractérisée par le fait que** le rapport pondéral SOD(s)/inhibiteur(s) de la 5-lipoxygénase est de 2/1 lorsque le ou les inhibiteurs de la 5-lipoxygénase sont choisis parmi le NDGA et le Flurbiprofen et de 3/1 lorsque le ou les inhibiteurs de la 5-lipoxygénase sont choisis parmi les acides gras polyinsaturés de la série oméga-3 tels que définis à l'une quelconque des revendications 1 à 3.

10. Composition selon l'une quelconque des revendications précédentes, **caractérisée par le fait qu'**elle renferme en outre un ou plusieurs principes actifs additionnels.

11. Composition selon l'une quelconque des revendications précédentes, **caractérisée par le fait qu'**elle se présente sous la forme d'un kit comprenant au moins une composition (A) renfermant au moins un inhibiteur de la 5-LO tel que défini précédemment et au moins une composition (B) renfermant au moins une superoxyde dismutase, lesdites compositions (A) et (B) étant destinées à être mélangées ensemble au moment de l'emploi.

12. Composition selon l'une quelconque des revendications précédentes, **caractérisée par le fait qu'**elle est destinée à être administrée par voie orale et se présente sous la forme de comprimés, de gélules, de solutions ou suspensions buvables, de tablettes, ou sous toute autre forme appropriée à ce mode d'administration.

13. Produit alimentaire **caractérisé par le fait qu'**il renferme, à titre d'ingrédient, au moins une composition telle que définie à l'une quelconque des revendications 1 à 10.

14. Utilisation d'au moins une composition telle que définie à l'une quelconque des revendications 1 à 12, à titre de médicament.

15. Utilisation selon la revendication 14, **caractérisée par le fait que** le médicament est destiné à la prévention et/ou au traitement des états inflammatoires chroniques, d'origine allergique ou non.

16. Utilisation d'au moins une composition telle que définie à l'une quelconque des revendications 1 à 12, à titre de produit diététique.

17. Utilisation selon la revendication 16, **caractérisée par le fait que** ledit produit diététique est destiné à la prévention des états inflammatoires chroniques, d'origine allergique ou non.

18. Utilisation d'au moins une composition telle que définie à l'une quelconque des revendications 1 à 12, pour la préparation d'un médicament destiné à la prévention et/ou au traitement des états inflammatoires chroniques d'origine allergique ou non.

19. Utilisation d'au moins une composition telle que définie à l'une quelconque des revendications 1 à 12, pour la préparation d'un produit diététique ou nutraceutique destiné à la prévention des états inflammatoires chroniques d'origine allergique ou non.

## Claims

1. Pharmaceutical or dietetic composition, **characterised in that** it comprises, in a pharmaceutically or dieteticcally acceptable support:
- at least one 5-lipoxygenase (5-LO) inhibitor selected from the group consisting of nordihydroguaiaretic acid (NDGA), the racemate of α-methyl-2-fluoro-[1,1'-biphenyl]-4-acetic acid (flurbiprofen) and omega-3 polyunsaturated fatty acids containing 18 or 20 carbon atoms and at least 3 unsaturated bonds, and
- at least one superoxide dismutase (SOD);
said composition being free of pyrimidine 3-oxide derivatives.

2. Composition according to claim 1, **characterised in that** the omega-3 polyunsaturated fatty acids are selected from linolenic acid (C18; n-3), 5,8,11,14-eicosatetraynoic acid, eicosapentaenoic acid, eicosatriynoic acid and mixtures thereof.

3. Composition according to claim 2, **characterised in that** it comprises a mixture of linolenic acid, 5,8,11,14-eicosatetraynoic acid and eicosapentaenoic acid.

4. Composition according to any one of the preceding claims, **characterised in that** the SODs are selected from SODs of animal, plant and bacterial origin and recombinant SODs of human, animal or plant origin.

5. Composition according to any one of the preceding claims, **characterised in that** the SOD or SODs are combined with at least one vector capable of facilitating the transmembrane passage of the SOD or SODs.

6. Composition according to claim 5, **characterised in that** said vector is selected from prolamines of plant origin, ceramides and polypeptides rich in arginine and/or in lysine.

7. Composition according to claim 6, **characterised in that** the plant prolamines are selected from the prolamines of wheat (gliadin), corn (zein) and rice.

8. Composition according to any one of the preceding claims, **characterised in that** the weight ratio SOD(s)/5-lipoxygenase inhibitor(s) is from 5/1 to 1/1 when the 5-lipoxygenase inhibitor or inhibitors are selected from NDGA and flurbiprofen and from 7/1 to 1/1 when the 5-lipoxygenase inhibitor or inhibitors are selected from omega-3 polyunsaturated fatty acids as defined in any one of claims 1 to 3.

9. Composition according to claim 8, **characterised in that** the weight ratio SOD(s)/5-lipoxygenase inhibitor(s) is 2/1 when the 5-lipoxygenase inhibitor or inhibitors are selected from NDGA and flurbiprofen and 3/1 when the 5-lipoxygenase inhibitor or inhibitors are selected from omega-3 polyunsaturated fatty acids as defined in any one of claims 1 to 3.

10. Composition according to any one of the preceding claims, **characterised in that** it further comprises one or more additional active ingredients.

11. Composition according to any one of the preceding claims, **characterised in that** it is in the form of a kit containing at least one composition (A) comprising at least one 5-LO inhibitor as defined hereinbefore and at least one composition (B) comprising at least one superoxide dismutase, said compositions (A) and (B) being intended to be mixed together at the time of use.

12. Composition according to any one of the preceding claims, **characterised in that** it is to be administered orally and is in the form of tablets, gelatin capsules, drinkable solutions or suspensions, lozenges or in any other form suitable for that mode of administration.

13. Food product, **characterised in that** it comprises, as an ingredient, at least one composition as defined in any one of claims 1 to 10.

14. Use of at least one composition as defined in any one of claims 1 to 12 as a medicament.

15. Use according to claim 14, **characterised in that** the medicament is for the prevention and/or treatment of chronic inflammatory conditions which may or may not be of allergic origin.

16. Use of at least one composition as defined in any one of claims 1 to 12 as a dietetic product.

17. Use according to claim 16, **characterised in that** said dietetic product is for the prevention of chronic inflammatory conditions which may or may not be of allergic origin.

18. Use of at least one composition as defined in any one of claims 1 to 12 in the preparation of a medicament for the prevention and/or treatment of chronic inflammatory conditions which may or may not be of allergic origin.

19. Use of at least one composition as defined in any one of claims 1 to 12 in the preparation of a dietetic or nutraceutical product for the prevention of chronic inflammatory conditions which may or may not be of allergic origin.

## Patentansprüche

1. Pharmazeutische oder diätetische Zusammensetzung, **gekennzeichnet durch** die Tatsache, dass sie, in einem pharmazeutisch oder diätetisch geeigneten Träger, umschließt:
- mindestens einen Inhibitor von 5-Lipoxygenase, ausgewählt aus der Gruppe gebildet aus Nordihydroguajaretsäure (NDGA) dem Racemat von α-Methyl 2-Fluoro [1,1'-Biphänyl]-4-Acetyl-Säure (Flurbiprofen) und den mehrfach ungesättigten Fettsäuren der Serie Omega-3, umfassend 18 oder 20 Kohlenstoffatome oder mindestens drei ungesättigte Bindungen, und
- mindestens eine Superoxiddismutase;
wobei die besagte Verbindung frei von Derivaten von Pyrimidin-3-Oxid ist.

2. Zusammensetzung gemäß dem Anspruch 1, **gekennzeichnet durch** die Tatsache, dass die mehrfach ungesättigten Fettsäuren der Serie Omega-3 ausgewählt sind aus Linolensäure (C18; n-3), aus 5,8,11,14-Eikosatetraenoesäure, aus Eikosapentaenoesäure, aus Eikosatrienoesäure und aus deren Gemische.

3. Zusammensetzung gemäß dem Anspruch 2, **gekennzeichnet durch** den Umstand, dass sie ein Gemisch aus Linolensäure, aus 5,8,11,14-Eikosatetraenoesäure und aus Eikosapentaenoesäure umschließt.

4. Zusammensetzung gemäß einem beliebigen der vorhergehenden Ansprüche, **gekennzeichnet durch** den Umstand, dass die SODs ausgewählt sind aus den SODs tierischer, pflanzlicher, bakterieller Herkunft und aus Rekombinanten SODs menschlicher, tierischer oder pflanzlicher Herkunft.

5. Zusammensetzung gemäß einem beliebigen der vorhergehenden Ansprüche, **gekennzeichnet durch** die Tatsache, dass das oder die SODs an mindestens einen Vektor gebunden sind, welcher geeignet ist, den Transmembranübergang der SODs zu erleichtern.

6. Zusammensetzung nach dem Anspruch 5, **gekennzeichnet durch** den Umstand, dass der besagte Vektor ausgewählt ist aus den Prolaminen pflanzlicher Herkunft, den Zeramiden und den Polypeptiden, welche reich an Arginin und/oder Lysin sind.

7. Zusammensetzung nach dem Anspruch 6, **gekennzeichnet durch** den Umstand, dass die pflanzlichen Prolamine ausgewählt sind aus den Prolaminen aus Getreide (Glyadin), von Mais (Zein) und von Reis.

8. Zusammensetzung nach einem beliebigen der vorhergehenden Ansprüche, **gekennzeichnet durch** den Umstand, dass das Gewichtsverhältnis SOD(s)/Inhibitor(en) von 5-Lipoxygenase zwischen 5/1 und 1/1 eingeschlossen ist, wenn der oder die Inhibitoren der 5-Lipoxygenase ausgewählt sind aus NDGA und Flurbiprofen, und zwischen 7/1 und 1/1, wenn der oder die Inhibitoren der 5-Lipoxygenase ausgewählt sind aus den mehrfach ungesättigten Fettsäuren der Serie Omega-3, wie beschrieben in einem der Ansprüche 1 bis 3.

9. Zusammensetzung nach dem Anspruch 8, **gekennzeichnet durch** den Umstand, dass das Gewichtsverhältnis SOD(s)/Inhibitor(en) der 5-Lipoxygenase 2/1 ist, wenn der oder die Inhibitoren der 5-Lipoxygenase ausgewählt sind aus NDGA und Flurbiprofen, und 3/1 ist, wenn der oder die Inhibitoren der 5-Liipoxygenase ausgewählt sind aus der Serie der mehrfach ungesättigten Fettsäuren der Serie Omega-3, wie diese in einem der Ansprüche 1 bis 3 beschrieben sind.

10. Zusammensetzung nach einem beliebigen der vorhergehenden Ansprüche, **gekennzeichnet durch** den Umstand, dass sie außerdem einen oder mehrere zusätzliche Wirkstoffe umschließt.

11. Zusammensetzung nach einem beliebigen der vorhergehenden Ansprüche, **gekennzeichnet durch** den Umstand, dass sie in Form eines Baukastens vorliegt, umschließend mindestens eine Zusammensetzung (A), welche mindestens einen Inhibitor der 5-LO, wie vorhergehend beschrieben, umschließt, und mindestens eine Verbindung (B), welche mindestens eine Superoxiddismutase umschließt, wobei die besagten Zusammensetzungen (A) und (B) dazu vorgesehen sind, im Augenblick der Verwendung vermischt zu werden.

12. Verbindung gemäß einem beliebigen der vorhergehenden Ansprüche, **gekennzeichnet durch** den Umstand, dass sie vorgesehen ist, um auf oralem Wege verabreicht zu werden und in Form von Pastillen, von Kapseln, von Lösungen oder trinkbaren Suspensionen, von Tabletten, oder in jeder anderen für diese Art der Verabreichung geeigneten Form vorliegt.

13. Lebensmittel, **gekennzeichnet durch** den Umstand, dass es als Bestandteil mindestens eine Zusammensetzung, wie sie **durch** einen beliebigen der Patentansprüche 1 bis 10 beschrieben ist, umschließt.

14. Verwendung mindestens einer Zusammensetzung, wie sie durch einen beliebigen der Patentansprüche 1 bis 12 beschrieben ist, als Arzneimittel.

15. Verwendung gemäß dem Anspruch 14, **gekennzeichnet durch** den Umstand, dass das Arzneimittel vorgesehen ist für die Vorbeugung vor und/oder die Behandlung von chronischen entzündlichen Zuständen, allergischen Ursprungs oder nicht, vorgesehen ist.

16. Verwendung mindestens einer Zusammensetzung wie sie durch einen beliebigen der Patentansprüche 1 bis 12 beschrieben ist, als diätetisches Erzeugnis.

17. Verwendung gemäß dem Patentanspruch 16, **gekennzeichnet durch** den Umstand, dass das besagte diätetische Erzeugnis vorgesehen ist für die Vorbeugung gegen chronische entzündliche Zustände, allergischer Herkunft oder nicht.

18. Verwendung mindestens einer Zusammensetzung, wie diese durch einen beliebigen der Patentansprüche 1 bis 12 beschrieben ist, für die Herstellung eines Arzneimittels für die Vorbeugung gegen und/oder Behandlung von chronischen entzündlichen Zuständen allergischer Herkunft oder nicht.

19. Verwendung mindestens einer Zusammensetzung, wie diese durch einen beliebigen der Patentansprüche 1 bis 12 beschrieben ist, für die Herstellung eines diätetischen Erzeugnisses oder Nahrungsmittels, welches für die Vorbeugung gegen chronische entzündliche Zustände allergischer Herkunft vorgesehen ist oder nicht.
